# EUROPEAN PATENT APPLICATION

(11) **EP 3 090 684 A1**
(43) Date of publication of application: **09.11.2016**
(21) Application number: 15167028.8
(22) Date of filing: 08.05.2015
(51) Int. Cl.: A61B 5/11, G01C 22/00

(54) **PEDOMETER AND METHOD FOR ANALYZING MOTION DATA**

(71) Applicant: The Swatch Group Research and Development Ltd., 2074 Marin (CH)
(72) Inventor: Fasel, Benedikt, 1022 Chavannes (CH); Duc, Cyntia, 1004 Lausanne (CH); Dadashi, Farzin, 1004 Lausanne (CH); Aminian, Kamiar, 1814 La Tour-de-Peilz (CH)
(74) Representative: Gilligmann, Benoît Philippe

(57) **Abstract**

The present invention relates to a pedometer and a method for analyzing motion data, wherein said method comprises the steps of:
- measuring motion data (D) of a user's upper body part by a motion sensor (12) attached to the wrist,
- transferring the motion data (D) into frequency data (FD),
- estimating a cadence (EC) on the basis of the frequency data (FD),
- detecting a stride motion at least on the basis of the frequency data (FD) or motion data (D) and on the basis of the estimated cadence (EC).

## Description

### Field of the invention

The present invention relates to the field of analyzing motion data for tracking and determining locomotion and strides of a user equipped with a pedometer. In particular the invention relates to pedometer applications and devices that are attachable to a wrist of a user.

### Background of the invention

Walking is one of the main activities of daily life of persons. There is a great interest to characterize the walking and/or running activity and to determine instantaneous velocity and traveled distance within a given time interval, such like travelled distance per day. In principle it is possible to monitor the walking activity of a person by using a motion detector attached to its wrist. Such motion detectors, typically comprising an accelerometer, may be worn like a watch. For estimating a walking and/or running velocity as well as for determining whether a person is actually in a state of rest or walking it is necessary to analyze the motion data obtainable from the motion detector in a rather sophisticated way.

Measured accelerations cannot be simply integrated because of sensor drift and arm movement artifacts. Moreover, counting of the total number of steps and multiplying the counted steps by a person-specific coefficient is not very practical as it requires a person-specific calibration and a rather elaborate training and learning of the pedometer. Moreover, commonly known techniques do not provide an instantaneous estimation or calculation of a walking velocity with high accuracy. It is therefore an object of the present invention to provide a method for analyzing motion data, a respective computer program as well as a pedometer device providing a rather instantaneous and precise estimation or calculation of a walking velocity by exclusively analyzing data obtainable from a motion sensor attachable to the wrist of a person.

It is a particular object to provide a method for analyzing motion data which is rather robust with regard to perturbations and which provides precise data about stride frequency and velocity without the necessity of elaborate calibration procedures.

Document US 2013/0085711 A1 discloses for instance a method for detecting steps at an electronic device, wherein the method comprises a threshold filtering of a motion data signal based on a moving average of the motion data signal to detect crossings of the motion data signal about the moving average; and detecting steps based on the detected crossings.

In practical implementation it is already rather difficult and sophisticated to precisely determine whether a person wearing a motion sensor at its wrist is actually standing still or is in a state of walking or running.

It is another goal of the present invention to provide pedometer applications and devices that do not require to be attached to a user's foot anymore.

### Summary of the invention

In a first aspect the invention relates to a method of analyzing motion data, and in particular to analyze motion data being characteristic of a state of walking or running of a person.

In a first step the method provides measuring of motion data of a user's upper body part by means of a motion sensor, which motion sensor is attached to any suitable upper body part of a user, such like a user's head, neck, arm, wrist, torso, waistline or hip. The motion data captured over a predefined time interval is transferred into respective frequency data. For instance, the motion data captured in a time domain over a predefined time interval is transferred into the frequency domain. Typically, transferring of the motion data into the frequency data implies calculating of a fast Fourier transform (FFT) of the motion data. Based on the frequency data a cadence is estimated. The estimated cadence is an initial guess or estimation of a cadence if the person was in a state of walking or running.

In order to calculate a velocity of the person on the basis of the captured motion data the method further comprises the step of detecting a stride motion. Detection of the stride motion implies to distinguish between a state of walking or running and a state in which the person is standing still. Distinguishing between a walking state and a rest position is rather difficult since a person may swing an arm or may rotate about its own longitudinal axis without substantially moving relative to the ground.

The method for analyzing the motion data and the detection of a stride motion is conducted at least on the basis of the frequency data and on the estimated cadence. The estimated cadence may reflect a locomotion probability inherently indicating whether a person is in a rest position or is in a state of walking or running. If the person is in a state of walking or running the cadence estimated on the basis of the frequency data will be typically within a typical range, e.g. between 0.5 Hz and 1.5 Hz for walking. Estimating a cadence below or above such a typical frequency interval may be a strong indication that the estimated cadence is somewhat unreasonable.

In this case and depending on the magnitude of the difference between the estimated cadence and a predefined reasonable cadence interval a rather good and precise distinction can be made whether the person is in a rest position or in a state of walking or running. The precision of the stride motion detection can be even enhanced when the estimated cadence is evaluated and processed in combination with the motion data, the frequency data and/or with at least one or several further parameters derived from the motion data or frequency data.

It is even conceivable that the detection of the stride motion is based on a parameter derived on the basis of the motion data and on the basis of a parameter derived from the frequency data. Alternatively, it may be already sufficient when the estimated cadence is just compared or related to one further parameter derived from the frequency data or motion data.

Estimating a cadence for the purpose of stride motion detection is also rather useful for a determination or calculation of a stride frequency. Hence, one and the same calculating methods or routines for estimating the cadence may be used several times, namely for detecting of a stride motion as well as for quantitatively determining a cadence or stride frequency.

According to a further embodiment, detecting the stride motion further includes determining a signal energy on the basis of the frequency data. The signal energy may be derived from the fast Fourier transform of the motion data. The signal energy is a direct indication of the total motion energy released by the person. In a current state of motion rather high signal energy is indicative of a state of walking or running whereas a comparatively low signal energy may indicate that the person is non-moving and is hence in a rest position.

According to another embodiment detection of the stride motion further includes determining of a temporal variation of the spectral distribution of the frequency data. Here, an abrupt change in the spectral distribution of the frequency data may indicate that the state of motion of the person of interest actually changes. If a person is in a rest position and starts to walk the respective change of the state of motion will be immediately apparent as a temporal variation of the spectral distribution of the frequency data.

While the person is walking the spectral distribution of the frequency data may be somewhat constant. When the person is in a rest position the spectral distribution of the frequency data of the measured motion data may be also rather constant. If the state of motion of the person is subject to a change, for instance if a person starts to walk or stops walking, such a modification of the state of motion will immediately reflect in temporal variations of the spectral distribution of the frequency data. By determining such temporal variations and by analyzing the spectral distribution of the frequency data rather precise information about an actual state and/or change of a state of motion can be obtained.

The detection of the stride motion can even be improved if the temporal variations of the spectral distribution of the frequency data is monitored and captured over time, so that actual spectral distributions can be compared to previously captured or calculated spectral distributions of the frequency data.

According to a further embodiment the stride motion detection further includes determining of a mean absolute deviation of the motion data during a predefined time interval. The mean absolute deviation of the motion data in comparison to the mean value of the motion data is also a direct indication that the state of motion of the person wearing the motion sensor actually changes.

According to a further embodiment detecting of the stride motion further includes calculating of a locomotion probability. The locomotion probability may be expressed in form of a number between 0 and 1. The locomotion probability is indicative on whether the person of interest is in a state of walking or running or whether said person is in a rest position. The locomotion probability is calculated by means of a naive Bayes probability model. Calculation on the basis of the naive Bayes probability model is based on the estimated cadence and on at least one of the aforementioned parameters: signal energy, variation of the spectral distribution and mean absolute deviation. Yet according to another variant embodiment, linear or non-linear regression models such as neural networks or support vector machines could also be used. The advantages of naive Bayes over other machine learning methods are for example its low complexity, robustness to over fitting and the computation of probabilities that can then be used for further improvement of classification accuracy.

It is generally conceivable, that at least two or that all three additional parameters obtainable from the frequency data or motion data are considered by the naive Bayes probability model in addition to the estimated cadence to determine a rather precise locomotion probability. The naive Bayes probability model is a statistical model and provides a technique for constructing classifiers. The naive Bayes approach assigns class labels to problem instances, represented as vectors of feature values, where the class labels are drawn from some finite sets. The naive Bayes model may not only represent a single algorithm for training classifiers but may include a family of different algorithms that are based on a common principle: all naive Bayes classifiers assume that the value of a particular feature is independent of the value of any other feature, given the class variable. Hence, the aforementioned parameters, estimated cadence, signal energy, temporal variation of the spectral distribution and the mean absolute deviation of the motion data may be assigned with a classifier specifying a probability that the respective parameter value corresponds to a stride motion or not.

The naive Bayes probability model may be based on the method of maximum likelihood. Hence, by means of the naive Bayes probability model a locomotion probability can be determined on the basis of the estimated cadence in combination with the signal energy, the variation of the spectral distribution and/or the mean absolute deviation of the motion data. The naive Bayes probability model may be trained on site, hence when implemented in a pedometer device. Alternatively, the naive Bayes model may be programmed prior to an implementation in a pedometer device.

According to a further embodiment detection of the stride motion includes a statistical evaluation of consecutive locomotion probabilities on the basis of a hidden Markov model (HMM). A Viterbi-based algorithm may then be used to judge whether a sequence of previously detected or calculated locomotion probabilities is reasonable. Viterbi-based algorithms will automatically update unlikely locomotion probability sequences to a more likely sequence, thus acting like a noise and low pass filter and improving classification results. For several reasons, the locomotion probability constantly calculated at discrete regular or irregular time intervals may provide rather large locomotion probabilities, thereby indicating that the person is actually in a state of walking or running. If for some reason a single or a few locomotion probabilities of a sequence of locomotion probabilities do not match with preceding or following locomotion probabilities the Viterbi-based algorithm may act as a kind of a filter. If the person is for instance in a state of walking and if the locomotion probabilities detected at five consecutive points of time is close to 1, a sixth locomotion probability is smaller than 0.5 and subsequent locomotion probabilities are again close to 1 the Viterbi-based algorithm will just ignore the sixth locomotion probability.

If for instance a person stops walking the locomotion probability will be comparatively high as long as the person is in the state of walking but the locomotion probability will almost abruptly drop down to a series of comparatively small values. If the Viterbi-based algorithm detects such a distinct step in a temporal sequence of calculated locomotion probabilities this is a clear indication that the person actually changed its state of motion. Typically, at least one or all of the following parameters, namely the estimated cadence, the signal energy, the temporal variation of the spectral distribution or the mean absolute deviation of the motion data are constantly determined and calculated over a predefined time interval or time window, of e.g. a few seconds, such like 5 or 6 seconds. This analysis window is constantly shifted in time by discrete regular or irregular steps, such like 1 second. In this way any consecutive second a new set of the above mentioned parameters is calculated, wherein the calculation is always based on the preceding time interval or time window, e.g. on the basis of the preceding 5 or 6 seconds.

Making use of such a shifting time window provides sufficient history for the analysis of the motion data to detect a stride motion, a locomotion probability as well as to determine almost instantaneously an actual stride frequency or walking velocity of the person. A rather sophisticated calibration with a specific person is no longer required.

According to another embodiment the estimated cadence is estimated by using a maximum harmonic matching scheme of the frequency data. For obtaining the estimated cadence the frequency data, hence a frequency spectrum of the measured motion data is subject to a frequency analysis. Here, various test frequencies homogeneously spread within an expectable range of the cadence, i.e. typically between 0.4 and 2Hz for someone walking, are applied and are further compared with peaks and with the spectral distribution of the frequency data. For this, a fundamental frequency and its higher harmonics are fitted into the frequency data.

That fundamental frequency that matches best with the frequency data is then selected or determined as the estimated cadence. For a determination of the best matching or best fitting the coefficients of those frequencies matching with a fundamental frequency and their respective higher harmonics are added up to a frequency-specific sum, denoted as a cadence likelihood. At the end the maximum of all sums, hence maximum cadence likelihood is determined and the respective frequency for which the maximum sum is obtained is selected as the estimated cadence.

For the maximum harmonic matching scheme it is of particular benefit, when the frequency data is interpolated in such a way that not only 64 or 128 frequency data but that a multiplicity thereof is subject to frequency analysis. In this way the precision of frequency determination can be substantially increased, thereby providing an improved stride motion detection as well as a rather accurate and improved stride frequency calculation.

According to another embodiment the method for analyzing motion data further comprises a step of unequivocally assigning the estimated cadence to a stride frequency. From the maximum harmonic matching of the frequency data there may arise two cadences or frequencies, wherein one frequency is a higher harmonic of the other frequency. If both conceivable frequencies are within a reasonable frequency range, e.g. between 0.5 Hz to 1.5 Hz it is rather difficult to determine a correct stride frequency only on the basis of the maximum harmonic matching scheme.

In order to unequivocally assign the estimated cadence to a stride frequency it is therefore suggested to compare a first estimated cadence which is estimated for a predefined time interval with a second estimated cadence that is estimated for a consecutive or preceding time interval. Depending on the comparison either the first or the second estimated cadence is then actually assigned to the stride frequency. If the first cadence is substantially different to the second cadence this is only reasonable when the person changed its state of motion. However, the changing of the state of motion is also reflected in the temporal sequence of the locomotion probability and is further reflected in the stride motion. Having knowledge of the actual stride motion the method may either select the first or the second cadence and assign one of the first and second cadences to the stride frequency.

Alternatively, the first estimated cadence may be compared with an intensity signal of the motion data or of the frequency data measured during the predefined time interval. For instance, a mean absolute deviation of the motion data or a maximum value of the motion data may be further indicative whether a first or a second cadence reflects the actual stride frequency.

According to another embodiment the method further comprises the step of calculating a velocity of the person on the basis of the stride frequency and/or on the basis of the estimated cadence. Calculating of the velocity is strongly correlated to the stride frequency of a person. Having determined the stride frequency allows calculating a respective velocity, i.e. walking or running velocity of the respective person when the step size or stride length is known. Calculating the velocity on the basis of the stride frequency is beneficial in terms of enabling a rather instantaneous velocity determination.

In another embodiment the velocity is calculated on the basis of at least two different cluster functions, each of which independently assigning at least two parameter values to a cluster-specific velocity. Typically, each one of the at least two cluster functions independently assigns at least two of a mean acceleration value of the motion data, a standard deviation thereof, a stride frequency or an estimated cadence and a person's height to a cluster-specific velocity model. For each cluster a different linear regression between input parameters and walking velocity is computed in a training step. This step is offline, during algorithm development. The model coefficients are then implemented into the watch and give one velocity per cluster for a given set of input parameters. Then the final velocity is found by a weighted average of the different "cluster velocities. In this way the cluster functions may comprise or provide a training database and a multi-parameter assignment between a cluster velocity model and at least one of a mean acceleration value parameter, a standard deviation parameter, a stride frequency parameter and a person's height parameter as well as further parameters by way of which the walking or running of a person may be generally characterized.

Each of the cluster functions independently and individually assign all these different walking parameters to a cluster velocity. The at least two different cluster functions are valid for different frequency intervals of the stride frequency. Only as an example, a first frequency interval may reach from 0.3 Hz to 0.65 Hz, a second interval may reach from 0.6 Hz to e.g. 1 Hz, a third interval may reach from 0.9 Hz to 1.2 Hz, and so on. The various cluster functions may provide parameter assignments in an overlapping fashion. So for each stride frequency each one of the at least two cluster functions provides a respective cluster velocity.

Having access to the at least two different cluster functions the method in a further aspect provides calculation of the velocity on the basis of the at least two cluster functions for a given height of a person and for a given, measured or estimated stride frequency. For this a weighted average of the at least two cluster velocities obtained on the basis of the given height and the given stride frequency are calculated by means of the at least two cluster functions. The at least two cluster functions provide a somewhat fuzzy logic approach to assign a stride frequency with a given height of a person to a person's velocity. In the present context the velocity is the velocity at which a person is moving relative to the ground.

The fuzzy logic approach is of particular benefit to derive and to determine a velocity of a person only and exclusively on the basis of the determined cadence even without determining or knowing the step size or stride length of the person. It is generally only necessary to provide information about the user's height. But even without information regarding the height of the person the fuzzy logic approach provides a velocity determination with excellent precision. Moreover, this approach is particularly well suited for low power applications as only a very limited set of multiplications and additions are required for estimating the person's velocity.

According to another aspect the invention also relates to a computer program for analyzing motion data, and in particular for determining a stride frequency and a velocity of a person wearing a motion sensor at an upper body part, i.e. no part pertaining to the foot or leg anymore. The computer program comprises computer program means for measuring, capturing and recording motion data of a user's upper body part, which motion data is obtainable from a motion sensor attached to the respective body part. Furthermore, the computer program comprises computer program means for transferring the motion data into frequency data. Typically, the computer program means are configured to calculate a Fourier transform of the motion data obtained during a predefined time interval, hence during a time window of predefined size.

In another embodiment the size of the time window and the time interval between two consecutive windows could be adapted based on the measured motion data. As such, the algorithm can adapt automatically to increase precision during walking and running periods and save energy during periods of low or no motion.

In addition, the computer program comprises computer program means for estimating a cadence based on the frequency data and further comprises computer program means for detecting a stride motion at least on the basis of the frequency data and on the basis of the estimated cadence.

In other words the computer program for analyzing motion data is configured to implement the method as described above in an electronic device comprising at least a processor, an input/output means and a memory. The computer program is particularly configured to execute the method as described above. Consequently, all features, method steps, advantages and effects obtainable via the method for analyzing motion data as described above are equally valid for the computer program and vice versa. The computer program is a software program product that can preferably be run on small appliances like smart phones or smart watches, and is designed to be compliant with as many devices and operating systems as possible.

In another aspect the invention also relates to a pedometer device for measuring a stride motion of a person. The device comprises a motion sensor attachable to a person's upper body part to measure motion data. The device is typically attachable to various upper body parts such like a user's head, neck, arm, wrist, torso, waistline or hip, i.e. a part remotely located with respect to the foot. The device is particularly suitable to detect a stride motion of the user and/or to determine cadence and velocity of the user. The device further comprises a frequency analyzer for transferring the motion data into frequency data. The device also comprises a cadence estimator for estimating a cadence based on the frequency data or the motion data and on the basis of the estimated cadence.

The pedometer device may further comprise a housing, which houses the motion sensor, the frequency analyzer, the cadence estimator and the stride detector as well as further electronic components to determine a velocity or walking velocity as well as to determine or to calculate a walking distance, and means for providing these information to the user, like display and/or hands and/or vibrations and/or other appropriate means. The pedometer device may comprise a bracelet and may be wearable at a user's wrist such like a wrist watch. In a further embodiment the pedometer device may be included or integrated into an electronic wrist watch or similar electronic device wearable at a user's wrist or attachable to other upper body parts.

In general the pedometer device is configured to execute the method for analyzing motion data as described above. Hence, any feature, advantage and effect mentioned in connection with the above described method for analyzing motion data equally apply to the pedometer device and vice versa. By means of the stride detector evaluating results of an estimated cadence and hence analyzing signals obtainable from the cadence estimator a very robust and precise distinction can be made whether a person wearing the pedometer device is in a rest position or is actually in a state of walking or running.

In another embodiment the pedometer device comprises at least one of a signal energy analyzer, a spectrum analyzer or a deviation calculator. The signal energy analyzer is configured to determine the signal energy of the frequency data. The spectrum analyzer is configured to determine temporal variations of the spectral distribution of the frequency data and the deviation calculator is configured to determine a mean absolute deviation of the motion data during a predefined time interval. By means of the signal energy analyzer, the spectrum analyzer and/or the deviation calculator further parameters can be evaluated and analyzed in addition to the estimate cadence in order to distinguish whether the person wearing the pedometer device is in a rest position or in a state of walking or running.

In another embodiment the stride detector further comprises a naive Bayes calculator to determine a locomotion probability on the basis of data provided by at least one of the cadence estimator, the spectrum analyzer, the signal energy analyzer and the deviation calculator. Based on at least two of the above mentioned parameters, namely estimated cadence, variations of the spectral distribution of the frequency data, signal energy of the frequency data and/or mean absolute deviation of the motion data the naive Bayes calculator is able to calculate a locomotion probability providing a number representing a probability whether the person wearing the pedometer device is actually in a state of walking or running or whether the person is in a rest position.

### Brief description of the drawings

In the following, preferred embodiments the present invention will be described by making reference to the drawings, in which:
- Fig. 1 shows a diagram of measured motion data over time with a first inspection window;
- Fig. 2 shows a diagram according to Fig. 1 with an inspection window shifted further in time by one discrete time step;
- Fig. 3 schematically shows a frequency diagram when the person is in a rest position;
- Fig. 4 shows a frequency diagram where the person makes various random movements;
- Fig. 5 shows a frequency diagram while the person is in a state of walking;
- Fig. 6 is illustrative of a flowchart for determining a locomotion probability;
- Fig. 7 shows a flowchart to detect a stride motion on the basis of the locomotion probability, and
- Fig. 8 shows a flowchart of a velocity calculation on the basis of the stride detection;
- Fig. 9 is illustrative of a frequency diagram when subject to a maximum harmonic matching procedure on the basis of a first test frequency;
- Fig. 10 shows the diagram according to Fig. 9 in addition with a second test frequency,
- Fig. 11 shows the diagrams according to Figs. 9 and 10, wherein a rather broad frequency range is subject to inspection;
- Fig. 12 shows a flowchart illustrating the maximum harmonic matching procedure;
- Fig. 13 shows a graph illustrating the determination of the estimated cadence on the basis of the maximum harmonic matching procedure;
- Fig 14 shows how additional interpolated frequency data are provided in order to simplify the processing of the frequency data while still ensuring good peak detection for the estimated cadence;
- Fig. 15 schematically shows calculation of a person's velocity on the basis of four different cluster functions;
- Fig. 16 schematically shows a block diagram of the pedometer device and
- Fig. 17 schematically shows a person wearing the pedometer device at a wrist.

### Detailed description

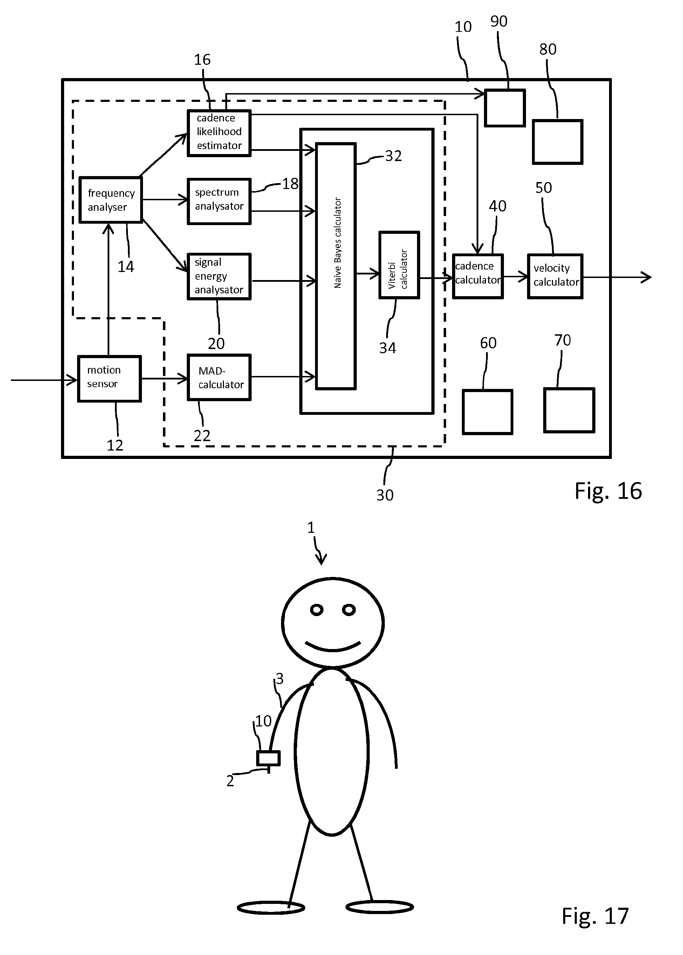

In Fig. 17 a person or user 1 is schematically illustrated wearing a pedometer device 10 at a wrist 2 of an arm 3. The pedometer device 10 is illustrated in Fig. 16 in more detail. It comprises a motion sensor 12, a stride detector 30, a cadence calculator 40 and a velocity calculator 50. In addition or optionally the pedometer device 10 may also comprise an input 60, typically in form of an input key, a touchscreen, a camera or a microphone. Furthermore and optionally the pedometer device 10 may comprise an output 70 to audibly or visually provide information to a user; alternatively, vibrating means could also be employed. Further optionally the pedometer device 10 may also comprise a communication unit 80 by way of which the pedometer device may communicate with another electronic device either by a wired or wireless data transmission line being not further illustrated here.

As it is further illustrated in Fig. 16 the pedometer device 10 optionally comprises a gait variability calculator 90. The input of the calculator 90 is connected with another output of the cadence likelihood estimator 16. In this way, the estimated cadence EC permanently and stepwise calculated during a measurement is provided to the calculator 90. The calculator 90 is configured to calculate the variability VAR of the sequence of estimated cadences EC over a predefined evaluation period T. The period T may be as long as a few tens of seconds.

For instance, the time evaluation period T may extend over a minute. During said period T the calculator 90 calculates a standard deviation of the cadence EC and provides gait variability as an output. The output of the calculator 90 may be presented to a user via the output 70 or via the communication unit 80 to another electronic device. Generally, the precision of the cadence estimation may be larger than 98% compared to a given reference measuring directly the leg movement. In addition also a stride-to-stride variability may be determined with a precision of even more than 99%.

The motion sensor 12 is typically implemented as an accelerometer configured to measure the acceleration in all three spatial directions x, y, z at which the pedometer device 10 is actually moved relative to the ground. Typically, the motion sensor 12 is configured to determine the root mean square of x² + y² + z², wherein x², y² and z² are representative of the square of the acceleration of the pedometer device 10 in the respective direction, x, y or z.

Although the motion sensor 12 could also encompass a gyroscope and a magnetometer, according to the preferred embodiment described therein only data captured by an accelerometer are processed in order to spare battery lifetime, since the other two sensor types (i.e. gyroscope and magnetometer) are significantly more demanding in terms of processing power. Moreover, comparative tests carried out have shown that the precision was only little affected when waiving these two sensors, so that a very good trade-off has been found in terms of processing power requirements and accuracy while keeping only an accelerometer for the sake of efficiency.

The stride detector 30 comprises various electronic units, which may be implemented in one or several electronic circuits or chips. Likewise also the cadence calculator 40 and the velocity calculator 50 may be integrated into one single chip or one integrated circuit together with the stride detector 30. Also the motion sensor 12 may be configured as an integral component of such an integrated circuit not further illustrated here. The stride detector 30 comprises a frequency analyzer 14. An input of the frequency analyzer 14 is connected with an output of the motion sensor 12. Typically, the frequency analyzer 14 is configured to transfer a motion signal or motion data D obtainable from the motion sensor 12 into the frequency domain.

Another output of the motion sensor 12 by way of which an acceleration signal over time can be provided is connected with an input of a mean absolute deviation calculator 22 (NB: it will be understood, however, that median absolute deviation or standard deviation could also be applied in the frame of the present invention. However, the mean absolute deviation is more efficient to compute as it does not involve sorting of data as for the median and does not involve squaring number or taking a square root). The mean absolute deviation (MAD) calculator 22 is a component of the stride detector 30. The stride detector 30 further comprises a cadence likelihood estimator 16, a spectrum analyzer 18 and a signal energy analyzer 20. The inputs of the cadence likelihood estimator 16, the spectrum analyzer 18 and the signal energy analyzer 20 are all connected with one or with different outputs of the frequency analyzer 14. In other words, the cadence likelihood estimator 16, the spectrum analyzer 18 and the signal energy analyzer 20 are operating in the frequency domain and are thus configured to analyze the frequency spectrum derivable from the motion data D of the motion sensor 12.

The stride detector 30 comprises a naive Bayes calculator 32 and a Viterbi calculator 34. The naive Bayes calculator 32 and the Viterbi calculator 34 are arranged in series. Hence, an output of the naive Bayes calculator 32 is provided to an input of the Viterbi calculator 34. The naive Bayes calculator 32 comprises various inputs to receive signals from the cadence likelihood estimator 16, the spectrum analyzer 18, the signal energy analyzer 20 and from the mean absolute deviation calculator 22 of the motion data. The naive Bayes calculator 32 or naive Bayes classifier is operable to process the parameters obtainable from the cadence likelihood estimator 16, the spectrum analyzer 18, the signal energy analyzer 20 and the MAD calculator 22 in order to calculate a locomotion probability LP.

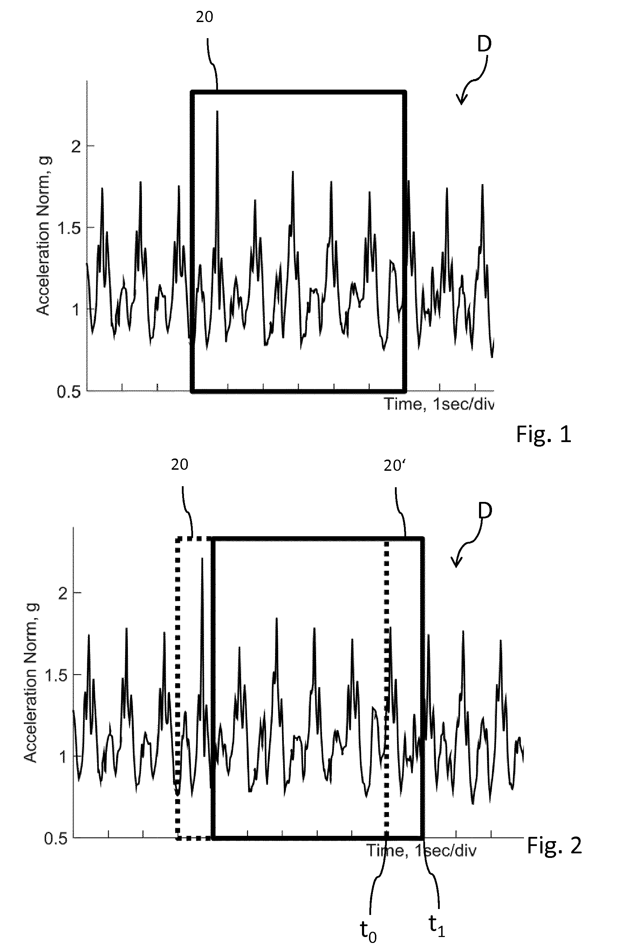

The locomotion probability LP represents a probability whether the data obtainable from the motion sensor 12 represents a rest position of the person 1 or a state of walking or running. For instance, the locomotion probability LP is a value between 0 and 1. Typically, the motion data D, in particular an acceleration of a user's wrist 2 is measured by a tri-axial accelerometer sampled at a rate of e.g. 20 Hz. The motion data D is permanently captured and recorded by means of an electric memory. In Fig. 1 a motion data D measured over a particular time interval is illustrated. While the motion data D is constantly measured the method for analyzing the motion data analyzes a predefined time window 20 that moves in discrete regular or irregular steps as the measurement time continues. For instance, the time window 20 may have a size of 6 seconds. Hence, at a given point of time t₀ the last 6 seconds prior to this particular point of time are actually analyzed in order to determine various parameters, such like estimated cadence EC, signal energy SE, temporal variation of the spectral distribution TVAR or mean absolute deviation MAD of the motion data.

As the measurement continues the time window 20' jumps discrete steps of e.g. 1 second as it is apparent from a comparison of Figs. 1 and 2. A time window 20 as indicated in the recorded motion data D of Fig. 1 has moved one second further in time and then represents a time shifted time window 20' at a time t₁ as shown in Fig. 2. There, the time window 20' is offset by 1 second from the time window 20 as shown in Fig. 1. For each time window 20, 20' parameters like estimated cadence EC, signal energy SE, temporal variation of the spectral distribution TVAR or mean absolute deviation MAD are permanently and instantly calculated.

In this way also for each discrete time step a locomotion probability LP is calculated at the output of the naive Bayes calculator 32. The Viterbi calculator 34 analyzes a temporal sequence of locomotion probabilities LP at the various discrete time steps t₀, t₁. The Viterbi calculator 34 compares an actual locomotion probability LP measured at a point of time t₁ with at least one previous locomotion probability LP obtained at a previous time, for instance at a time t₀. Moreover, the Viterbi calculator 34 is operable to analyze a sequence of locomotion probabilities LP calculated as a consecutive series of discrete time steps.

If for instance the locomotion probability LP is above a predefined threshold, thereby indicating that the person 1 is in a state of walking or running the Viterbi calculator 34 assumes that a proceeding locomotion probability LP will also reflect a state of walking or running. If for instance a proceeding locomotion probability calculated at a discrete time t + 1 is below a predefined locomotion threshold the Viterbi calculator just waits until receiving a next locomotion probability at a proceeding time step time t + 2. If this next locomotion probability is also below a predefined locomotion threshold the Viterbi calculator 34 may determine that a previous state of walking or running has changed to a rest position.

However, if the next locomotion probability LP is again above the locomotion threshold the Viterbi calculator 34 just neglects a single locomotion probability strongly deviating from a sequence of locomotion probabilities. In this way the stride detector 30 is rather robust against perturbations which may otherwise have a rather direct or instant impact on the locomotion probability LP.

An output of the stride detector 30 is hence provided by the output of the Viterbi calculator 34. The output of the Viterbi calculator 34 represents a stride motion and is hence indicative on whether the person carrying the motion sensor 12 is in a rest position or in a state of walking or running.

Once the stride detector 30 has detected a state of walking or running the cadence calculator 40 is operable to determine or to calculate not only an estimated cadence EC but to precisely determine a stride frequency SF. For this the cadence calculator 40 may be coupled to the cadence likelihood estimator 16. Calculation of the velocity V of the person 1 may be simply based on an unequivocal selection or assignment of one of a plurality of estimated cadences EC₁ or EC₂ to a stride frequency SF. For this the cadence calculator 40 may compare a first estimated cadence EC₁ estimated for a predefined time interval 20 with a second estimated cadence EC₂ estimated for a consecutive or preceding time interval 20'. If the comparison of EC₁ and EC₂ determines a rather large difference between the two estimated cadences this may reflect a change of the state of motion.

Such a modification of a state of motion should also be detectable and should also reflect in the signal energy SE or in the temporal variation of the spectral distribution TVAR or the absolute deviation MAD of the motion data D. If the further analysis of these parameters SE, TVAR or MAD reveals that the state of motion has not changed between the time intervals 20 and 20' the actually estimated cadence EC₁ may be discarded for the benefit of a previously estimated cadence EC₀, e.g. estimated for a time interval t₋₁ preceding the time interval 20.

For the precise calculation of the cadence it is also conceivable that the first estimated cadence EC₁ is compared with an intensity signal IS of the motion data D or of the frequency data FD measured during the predefined time interval 20. A quantitative comparison of such an intensity signal IS with the first estimated cadence EC₁ may provide an indication that for determining of a stride frequency SF the estimated cadence EC₁ has to be multiplied by a predefined factor, e.g. by a factor 0.5 or by a factor 2. Typically, the unequivocal assignment of the estimated cadence EC to a realistic stride frequency SF of the user 1 implies a selection of the fundamental frequency or of higher harmonics of the estimated cadence.

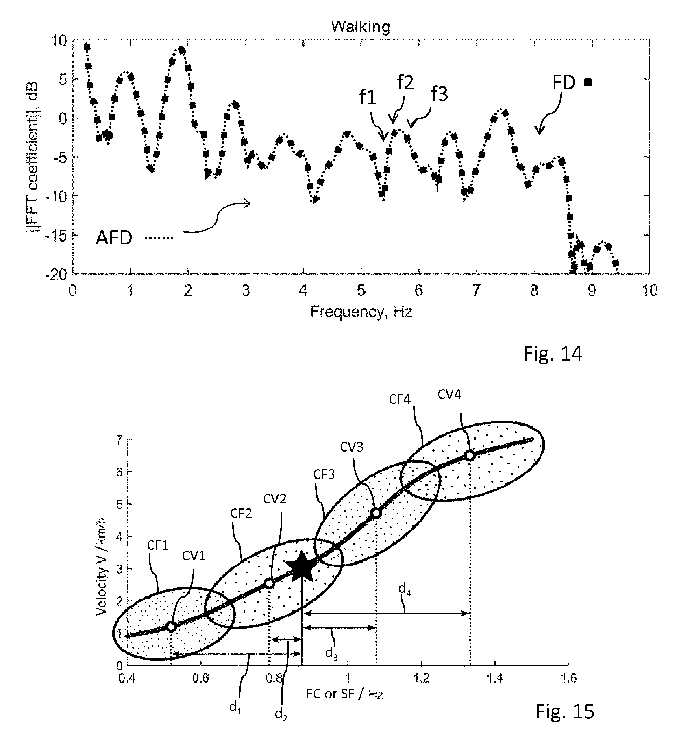

Once the stride frequency SF has been calculated by the cadence calculator 40 a velocity V of the user 1 is determined by means of the velocity calculator 50. Calculation of the velocity is further illustrated in Fig. 14. There, a diagram of a velocity V versus determined or calculated cadence is illustrated. The relation between the velocity V of the person 1 and the cadence or stride frequency SF is modeled by at least two different cluster functions CF1, CF2 and so on. In the present embodiment there are provided four different cluster functions CF1, CF2, CF3, CF4. Any cluster function CF1, CF2, CF3, CF4 independently assigns at least two of the parameters of a mean acceleration value MAV of the motion data, a standard deviation SDEV thereof, a stride frequency SF or estimated cadence EC and a person's height H to a cluster velocity CV1, CV2, CV3, CV4.

According to a preferred non-illustrated embodiment, use of barometric pressure as an additional feature could be included to adjust speed values since the speed is significantly lower for steep uphill and downhill sections as compared the ones observed on a flat ground. A rapid increase or decrease of measured pressure values would then indicate that the trajectory of the user is along a steep downhill, or respectively uphill track.

Any of the cluster functions CF1, CF2, CF3, CF4 provides an assignment between the measured stride frequency SF to a velocity of the person 1 for a given interval of the stride frequency SF. For determining a velocity V of the person, the given height H of the person 1 and a measured stride frequency SF are used to calculate various cluster velocities CV1, CV2, CV3, CV4. For determining the velocity V a weighted average of the at least two cluster velocities CV1, CV2 obtained from the at least two cluster functions CF1, CF2 is calculated. In the present embodiment the four cluster velocities CV1, CV2, CV3, CV4 of the four cluster functions CF1, CF2, CF3, CF4 are calculated and the velocity V is determined as a weighted average of the four independently determined cluster velocities CV1, CV2, CV3 and CV4.

The individual cluster velocities CV1, CV2, CV3, CV4 are weighted by the distance of the center of each cluster function CF1, CF2, CF3, CF4 to the measured stride frequency SF. As shown in Fig. 15, the center of the cluster function CF1 is separated by a distance d₁ from the stride frequency SF of interest. The center of the cluster function CF2 is separated from the stride frequency SF by a distance d₂, the center of the cluster function CF3 is separated from the stride frequency SF by a distance d₃ and the center of the cluster function CF4 is separated by a distance d₄ from the stride frequency SF. The actual velocity V of the person 1 V is obtained by a weighed sum of CV₁, ... CV₄ inversely proportional to their respective distances d₁, ... d₄.

In this way a typical and non-linear behavior between the velocity V and the stride frequency SF can be modeled by numerous cluster functions CF1, CF2, CF3, CF4, each of which providing good and reliable cluster velocities in a particular multi-parameter-space at least composed of the stride frequency, the signal energy, temporal variation of the spectral distribution and/or the mean absolute deviation of the motion data.

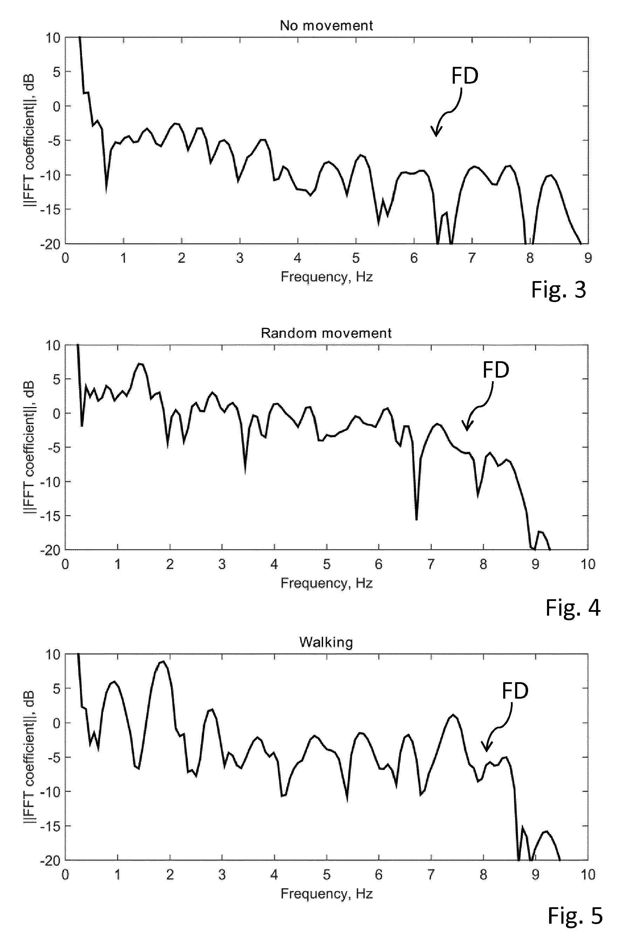

The diagram as shown in Figs. 3 and 4 are only exemplary and illustrate comparative frequency distributions of motion data D during a given or selected time window 20, 20' . The frequency spectrum as shown in Fig. 3 seems to be somewhat pseudo harmonic and comprises a rather low amplitude of <-5dB, but when applying the present method for analyzing motion data D a locomotion probability LP will be rather low.

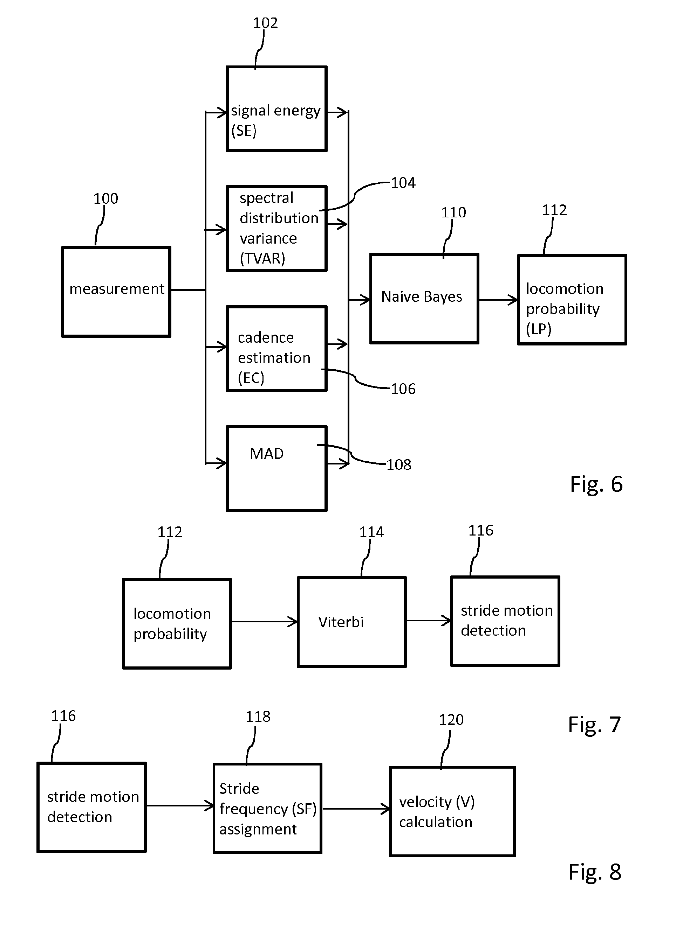

The spectral distribution as shown in Fig. 5 obtained during a state of walking is further analyzed by the present method as will be described in view of Figs. 6-14. In Fig. 6 a flowchart is given for determining a locomotion probability LP. In a first step 100 motion data D is extracted and captured on the basis of the electrical signals permanently obtained from the motion sensor 12. Thereafter, in steps 102, 104, 106, 108, various parameters are simultaneously or subsequently calculated on the basis of the motion data D in the time domain and/or on the basis of frequency data FD in the frequency domain. In step 102 a signal energy SE of the frequency data FD is calculated. In step 104 a temporal variation of the spectral distribution TVAR of the frequency data FD is calculated. In step 108 a mean absolute deviation MAD of the motion data D is calculated during the predefined time interval 20, i.e. during the predefined time window that precedes that present point of time.

In addition, also an estimated cadence EC is calculated in step 106 on the basis of the frequency data FD. The calculations of steps 102, 104, 106, 108 are all based on the motion data D or the frequency data FD of a given time window 20, 20'. The parameters SE, TVAR, EC and MAD are then evaluated and processed in step 110 by a naive Bayes probability model providing in step 112 a locomotion probability LP indicative on whether the person 1 is in a rest position or in a state of walking or running.

The locomotion probability LP is repeatedly calculated at any discrete time step as the time window 20 is shifted one time step further to a time window 20'. In the flowchart of Fig. 7 the sequence of locomotion probabilities LP is further analyzed by a Viterbi-based algorithm in step 114. There, consecutive locomotion probabilities LP obtained for consecutively propagating time windows 20, 20', and so on are evaluated in order to sort out or to filter unreasonable locomotion probabilities. The output of the Viterbi-based analysis conducted in step 114 is then provided in step 116 as the stride detection.

As further illustrated in Fig. 8 and once a state of walking or running has been determined in step 116 the cadence or a stride frequency SF is calculated or assigned in step 118. Based on the stride frequency SF and based on a given height of a person as well as based on optional further parameters, such like mean acceleration value MAV of the motion data D during the time window 20 and/or a standard deviation SDEV thereof a velocity V of the person 1 is calculated in step 120.

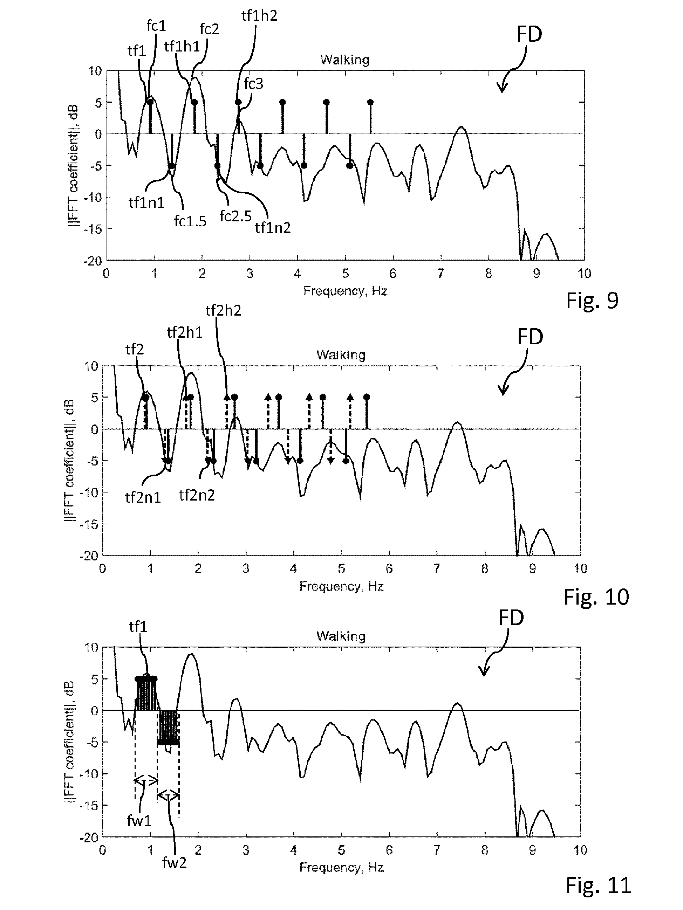

In the following reference is made to Figs 9-14. In Fig. 9 frequency data FD of motion data D captured during a predefined time window 20 is illustrated in the frequency domain. The frequency data FD as shown in Figs. 9-11 is obtained by a Fourier transformation, typically by a fast Fourier transformation of the captured motion data D as for instance shown in Figs. 1 and 2. For the frequency data FD obtained at a discrete time and representing a time window 20, of e.g. 6 or 5 seconds preceding the actual point of time the method of estimating a cadence EC starts with selecting of a first test frequency tf1 as a fundamental frequency and determining higher harmonic frequencies tf1 h1, tf1h2 thereof. In practice harmonic frequencies up to the third harmonic only are taken into account for processing purposes because the higher harmonics are not so much representative of any relevant signal values. As a result, this limitation simplifies the overall processing without deteriorating the results quality. Yet for further increasing the resolution sensitivity of the cadence estimator non-integer multiples of the fundamental frequency tf1 n1, tf1n2, as shown at the bottom of Fig. 9, can be selected as well.

In the example as illustrated in Fig. 9 a first test frequency tf1 slightly less than 1 Hz is selected. Then, higher harmonic frequencies tf1 h1, tf1h2, tf1 n1, and tf1n2 are calculated based on the first test frequency tf1. The higher harmonic frequencies tf1 h1, tf1h2, tf1 n1, and tf1n2 are also indicated in Fig. 9. The higher harmonic frequencies tf1 h1 and tf1h2 are slightly below 2 Hz and 3 Hz, respectively, whereby tf1 n1 and tf1n2 are located midways between tf1, tf1h1, and tf1h2 and show negative values.

In Fig. 10 selection of a second test frequency tf2 as a fundamental frequency is illustrated. Compared to the first test frequency tf1 the second test frequency tf2 is slightly lower. The slight difference between the first and the second test frequencies tf1, tf2 is particularly apparent in the higher harmonic frequencies tf2h1, tf2h2 in comparison to the first higher harmonic frequencies tf1 h1 and tf1h2. Similarly, non-integer multiples of the fundamental frequency tf2n1, tf2n2 are also represented.

Now, for each test frequency tfi with i representing an index for each available test frequency a cadence likelihood cli is calculated. For calculating a first cadence likelihood cl1 the frequency data FD at the first test frequency tf1 and its higher harmonic frequencies tf1h1, tf1h2, tf1 n1, tf1n2 are selected.. The cadence likelihood cl1 is then computed by summing up the frequency coefficients fc1, fc2 and fc3 of FD corresponding to tf1, tf1 h1, tf1h2, respectively, and subtracting therefrom the frequency coefficients fc1.5 and fc2.5 of FD corresponding to tf1 n1 and tf1n2, respectively. In the present case, the cadence likelihood equals fc1 - fc1.5 + fc2 - fc2.5 + fc3 shown on Fig. 9.

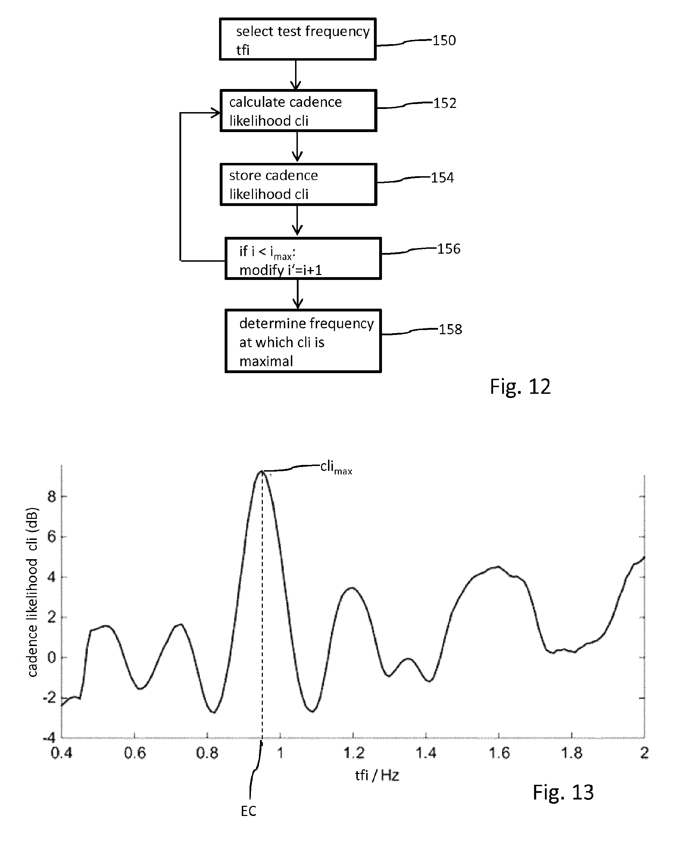

Respective cadence likelihood cli are then calculated for all test frequencies tfi, which are typically spread over the frequency domain between 0.4Hz and 2Hz, corresponding to the cadence that can be expected for a walking person. According to a preferred embodiment, the frequency resolution is chosen to be equal to 0.01 Hz between all subsequent test frequencies, in order to provide a granularity complying with product requirements. Once the calculation of the cadence likelihood cli has been obtained for all available test frequencies tfi, the cadence likelihood can and will be analyzed as a function of the test frequencies as illustrated in Fig. 13. Then, a maximum of all cadence likelihood cliₘₐₓ is determined. The frequency at which cliₘₐₓ is obtained is then defined as the estimated cadence EC. Apparently and as shown in Fig. 13 the maximum of the cadence likelihood cli is located close to 0.95 Hz.

The analysis of the relation of the cadence likelihood cli over the test frequencies tfi is of direct and further use for a determination of the stride motion of the person 1. In situations, wherein the maximum cadence likelihood only slightly distinguishes from a local maximum of the cadence likelihood it may be assumed, that there is no predominant fundamental frequency in the frequency data FD. This may be an indication that the person is actually not walking or running but is standing still but moves its arms.

It is apparent from Fig. 14, by means of an interpolation of available frequency data FD the horizontal resolution and hence the precision of determining a maximum cadence likelihood can be easily increased, without requiring to simultaneously increase the number of FFT points while performing the frequency data analysis, and hence comply with available CPU and volatile memory resources. In this way the estimated cadence as well as a stride frequency of a walking or running person can be calculated with arbitrarily high precision. Typically, for a given set of discrete frequencies f1, f2, f3 with frequency data FD the interpolation method provides additional frequency data AFD for frequencies located between the given frequencies f1, f2, f3. For instance, the additional frequency data AFD at these additional frequencies is calculated by a linear interpolation of the given set of frequency date FD. The resolution of the interpolated frequencies preferably matches the one of the test frequencies tfi in order to simplify the correlation operation performed in the frame of the harmonic matching scheme described here above in view of Figs 9-13. Therefore, the frequency data FD, typically compromising of 64 or 128 points, is interpolated at 0.01 Hz frequency steps, for example obtaining 1000 equally spaced points between 0 and 10Hz, schematically depicted as AFD in Fig. 14. According to a preferred embodiment the interpolation algorithm can be linear or nearest neighbor, in order to limit the necessary processing to a minimum. The frequency resolution could be easily adjusted and optimized to conform to other product requirements.

In Fig. 12 a flowchart is given illustrating the estimation of a cadence on the basis of the frequency data FD. In a first step 150 a test frequency tfi is selected. Thereafter, in step 152 a cadence likelihood cli is calculated as described above. Thereafter, in step 154 the calculated cadence likelihood cli is stored in a memory. Then, in step 156 it is evaluated whether the cadence likelihood cli has already been calculated for all available test frequencies tfi. If the index i is smaller than a predefined iₘₐₓ the index i is increased to i' e.g. by selecting the next discrete test frequency of the frequency data. Then, the method returns to step 152 and calculates a new cadence likelihood cli' on the basis of a further test frequency tfi' or tf(i +1). The loop of the steps 152, 154, 156 continues until all available test frequencies tfi have been subject to the harmonic matching scheme and until for each available test frequency tfi a cadence likelihood cpi has been determined and stored in the memory.

Thereafter, in step 158 a particular test frequency tfi is determined at which the cadence likelihood cli is maximal. Then, the respective test frequency tfi is assigned as the estimated cadence EC.

In Fig. 11 a further analysis of the frequency data FD is illustrated by way of which the previously estimated cadence EC is unequivocally assigned or recalculated to a stride frequency SF at which the person 1 actually walks. The above described method of calculating or determining the estimated cadence EC may be somewhat ambiguous. In practical situations a cadence during walking may range between 0.5 Hz to 1.4 Hz. In order to unequivocally determine a stride frequency on the basis of a potentially ambiguous estimated cadence the method for analyzing motion data may provide a comparison of a first and a second estimated cadence EC₁ and EC₂ that are estimated for consecutive time intervals, such like at different time windows 20, 20'. If the first estimated cadence EC1 determined for a first time window 20 strongly deviates from the second estimated cadence EC₂ determined or estimated for a consecutive time window 20' this is either an indication that the state of motion of the person 1 has changed between the time intervals 20, 20'.

Such a modification of the state of motion should be observable also by other parameters, such like signal energy SE, variation of the spectral distribution TVAR of the frequency data FD and/or on the basis of an analysis of the mean absolute deviation MAD of the motion data D. If the analysis of these further parameters reveals that the state of motion did not substantially change between the time windows 20, 20' then the estimated cadence EC₂ strongly deviating from the previously determined estimated cadence EC₁ will be discarded.

Alternatively or additionally, it is also conceivable that the estimated cadence EC is compared with an intensity signal IS of the motion data or of the frequency data, which intensity signal IS is measured during the predefined time interval or time window 20. The intensity signal IS, of the motion data D or frequency data FD may be obtained in many different ways. The intensity signal may equal the maximum of the standard deviation of the motion data. Typically and as observed in various tests the size or magnitude of the cadence is related to the intensity signal. By evaluating and analyzing the intensity signal IS derivable from motion data or frequency data an unequivocal assignment or recalculation of the estimated cadence EC can be conducted.

In this way it may be unequivocally determined whether the estimated cadence EC has to be multiplied by a predefined factor, e.g. by 0.5 or 2 in order to switch to a fundamental frequency or to a higher harmonic frequency that actually corresponds to the stride frequency at which the person 1 is actually walking.

Moreover and as particularly illustrated in Fig. 11, for the determination and calculation of a stride frequency SF it is also conceivable to analyze the slope of the peaks in the spectrum of the frequency data FD in direct vicinity to those test frequencies tf1 that are close to the estimated cadence EC. Hence, for the unequivocal determination of estimated cadences various frequency windows fw1, fw2 may be analyzed for unequivocal cadence estimation or stride frequency determination. In a preferred embodiment the width of both fw1 and fw2 can be selected between 0.1 Hz and 0.3Hz. This bandwidth value is optimized for a tradeoff between sufficient low pass filtering, low computation requirements, and reasonable frequency resolution. Increasing the bandwidth covers more of both the positive and negative peaks and is therefore - at a loss of frequency resolution - more robust in the case of noisy movement, reduce peak height and multiple small peaks. Therefore, the cadence can be determined more robustly.

### List of Reference numerals

- 1: person
- 2: wrist
- 3: arm
- 10: pedometer device
- 12: motion sensor
- 14: frequency analyzer
- 16: cadence estimator
- 18: spectrum analyzer
- 20: signal energy analyzer
- 22: mean absolute deviation calculator
- 30: stride detector
- 32: naive Bayes calculator
- 34: Viterbi calculator
- 40: cadence calculator
- 50: velocity calculator
- 60: input
- 70: output
- 80: communication unit
- 90: gait variability calculator
- 100: Measurement step
- 102: Signal energy computation step
- 104: Spectral distribution variance computation step
- 106: Cadence estimation computation step
- 108: Mean absolute deviation computation step
- 110: Naïve Bayes probability model processing
- 112: Yielding locomotion probability (LP)
- 114: Viterbi analysis
- 116: Stride motion detection step
- 118: Stride frequency (SF) assignment step
- 120: Velocity (V) calculation step
- 150: Step of selecting a test frequency *tfi*
- 152: Step of calculating a cadence likelihood *cli*
- 154: Step of storing a cadence likelihood *cli*
- 156: Incrementation step to the next discrete frequency
- 158: Step of determining the frequency for which the *cli* is maximal

- AFD: Additional frequency data
- D: Motion data
- CF1: First cluster function
- CV1: First cluster velocity
- CF2: Second cluster function
- CV2: Second cluster velocity
- CF3: Third cluster function
- CV3: Third cluster velocity
- CF4: Fourth cluster function
- CV4: Fourth cluster velocity
- EC: Estimated cadence
- EC1: First estimated cadence
- EC2: Second estimated cadence
- FD: Frequency data
- H: Height (of a person)
- LP: Locomotion probability
- MAD: Mean absolute deviation
- SDEV: Standard Deviation
- SE: Signal energy
- SF: Stride frequency
- T: Period
- TVAR: Spatial distribution
- V: Velocity
- VAR: Variability
- cli: Cadence likelihood
- f1,f2,f3: Set of discrete frequencies
- fc1: First frequency component
- fc1.5: First intermediate frequency component
- fc2: Second frequency component
- fc2.5: Second intermediate frequency component
- fc3: Third frequency component
- fw1: First frequency window
- fw2: Second frequency window
- tfi: Test frequency
- tf1: First test frequency
- tf1h1: First harmonic of the first test frequency
- tf1h2: Second harmonic of the first test frequency
- tf1n1: First non-integer harmonic of the first test frequency
- tf1n2: Second non-integer harmonic of the first test frequency
- tf2: Second test frequency
- tf2h1: First harmonic of the second test frequency
- tf2h2: Second harmonic of the second test frequency
- tf2n1: First non-integer harmonic of the first test frequency
- tf2n2: Second non-integer harmonic of the first test frequency

## Claims

1. A method for analyzing motion data, the method comprising the steps of:
- measuring motion data (D) of a user's upper body part by a motion sensor (12) attached to said upper body part,
- transferring the motion data (D) into frequency data (FD),
- estimating a cadence (EC) on the basis of the frequency data (FD),
- detecting a stride motion at least on the basis of the frequency data (FD) or motion data (D) and on the basis of the estimated cadence (EC).

2. The method according to claim 1, wherein detecting of the stride motion further includes determining of a signal energy (SE) on the basis of the frequency data (FD).

3. The method according to any one of the preceding claims, wherein detecting of the stride motion further includes determining of a temporal variation of the spectral distribution (TVAR) of the frequency data (FD).

4. The method according any one of the preceding claims, wherein detecting of the stride motion further includes determining of a mean absolute deviation (MAD) of the motion data (D) during a predefined time interval (20, 20').

5. The method according to any one of the preceding claims, wherein detecting of the stride motion includes calculating of a locomotion probability (LP), wherein the locomotion probability (LP) is calculated by means of a naive Bayes probability model on the basis of the estimated cadence (EC) and on the basis of at least one of the following parameters: signal energy (SE), variation of the spectral distribution (TVAR) and mean absolute deviation (MAD).

6. The method according to claim 5, wherein detecting of the stride motion includes a statistical evaluating of consecutive locomotion probabilities (LP) on the basis of a Hidden Markov Model and/or a Viterbi-based algorithm.

7. The method according to any one of the preceding claims, wherein the estimated cadence (EC) is estimated by using a maximum harmonic matching scheme of the frequency data (FD).

8. The method according to claim 7, further comprising a step of unequivocally assigning the estimated cadence (EC) to a stride frequency (SF) by:
- comparing a first estimated cadence (EC₁) estimated for a predefined time interval (20) with a second estimated cadence (EC₂) estimated for a consecutive or preceding time interval (20'), and/or
- comparing the first estimated cadence (EC₁) with an intensity signal of the motion data (D) or if the frequency Data (FD) measured during the predefined time interval (20, 20'), and
- assigning one of the first and second estimated cadences (EC₁, EC₂) to the stride frequency (SF) on the basis of the comparison or assigning the first estimated cadence (EC₁) multiplied by a predefined factor to the stride frequency (SF).

9. The method according to any one of the preceding claims 7 or 8, further comprising the step of calculating a velocity (V) on the basis of the stride frequency (SF) and/or on the basis of the estimated cadence (EC).

10. The method according to claim 9, wherein the velocity (V) is calculated on the basis of at least two different cluster functions (CF1, CF2) independently assigning at least two of a mean acceleration value (MAV) of the motion data (D), a standard deviation (SDEV) thereof, a stride frequency (SF) or estimated cadence (EC) and a person's height (H) to a cluster velocity (CV).

11. The method according to claim 10, wherein the velocity (V) is calculated for a given height (H) of a person (1) and for a given stride frequency (SF) on the basis of a weighted average of the at least two cluster velocities (CV) of the at least two cluster functions (CF1, CF2).

12. A computer program for analyzing motion data, arranged to carry out the steps of the method according to any of the previous claims 1 to 11, the computer program comprising:
- computer program means for measuring, capturing or recording motion data (D) obtainable from a motion sensor (12) attached to an upper body part of a person (1),
- computer program means for transferring the motion data (D) into frequency data (FD),
- computer program means for estimating a cadence (EC) based on the frequency data (FD),
- computer program means for detecting a stride motion at least on the basis of the frequency data (FD) or motion data (D) and on the basis of the estimated cadence (EC).

13. A pedometer device for measuring a stride motion of a person (1), suitable for executing the computer program of claim 12, the device comprising:
- a motion sensor (12) attachable to an upper body part of the person to measure motion data (D),
- a frequency analyzer (14) for transferring the motion data (D) into a frequency data (FD),
- a cadence estimator (16) for estimating a cadence (EC) based on the frequency data (FD), and
- a stride detector (30) for at least detecting a stride motion on the basis of the frequency data (FD) or motion data (D) and on the basis of the estimated cadence (EC).

14. The pedometer device according to claim 13, further comprising at least one of:
- a signal energy analyzer (20) to determine a signal energy (SE) of the frequency data (FD),
- a spectrum analyzer (18) to determine temporal variations of the spectral distribution (TVAR) of the frequency data (FD),
- a deviation calculator (22) to determine a mean absolute deviation (MAD) of the motion data (D) during a predefined time interval (20, 20').

15. The pedometer device according to claim 14, wherein the stride detector (30) comprises a naive Bayes calculator (32) to determine a locomotion probability (LP) on the basis of data or parameters provided by at least one of the cadence estimator (16), the spectrum analyzer (18), the signal energy analyzer (20) and the deviation calculator (22).

16. The pedometer device according to any of the claims 13 to 15, wherein said pedometer device is integrated into a wrist watch.
